# EUROPEAN PATENT APPLICATION

(11) **EP 4 279 065 A1**
(43) Date of publication of application: **22.11.2023**
(21) Application number: 22739108.3
(22) Date of filing: 14.01.2022
(51) Int. Cl.: A61K 9/06, A61K 47/14, A61K 45/00, A61K 47/10, A61K 47/12, A61K 31/635, A61K 31/445, A61K 47/44, A61K 47/28, A61K 47/26, A61P 29/00

(54) **SUSTAINED-RELEASE PREPARATION COMPOSITION**

(30) Priority: 14.01.2021 CN 202110051096
(71) Applicant: Nanjing Delova Biotech Co. Ltd., Nanjing, Jiangsu 211122 (CN)
(72) Inventor: LI, Ling, Nanjing, Jiangsu 211122 (CN); WU, Qu, Nanjing, Jiangsu 211122 (CN); JI, Yuanxin, Nanjing, Jiangsu 211122 (CN); WANG, Qingsong, Nanjing, Jiangsu 211122 (CN)
(74) Representative: Gille Hrabal Partnerschaftsgesellschaft mbB Patentanwälte
(86) International application number: PCT/CN2022/071928
(87) International publication number: WO 2022/152232

(57) **Abstract**

An oil gel pharmaceutical composition, comprising a liquid oil, a pharmaceutically acceptable gelator, a pharmaceutically acceptable stabilizer, and a pharmaceutically active component. The pharmaceutical composition is particularly suitable for a pharmaceutical preparation having anesthetic and analgesic activity, has good release duration and stability, can be used for injection and topical administration, has good patient tolerance and very few side effects.

## Description

The present application claims priority to Chinese Patent Application No. 202110051096.4 filed with China National Intellectual Property Administration on Jan. 14, 2021 and entitled "SUSTAINED-RELEASE FORMULATION COMPOSITION", which is incorporated herein by reference in its entirety.

### TECHNICAL FIELD

The present disclosure relates to the field of pharmaceutical formulations, and particularly relates to a sustained-release formulation composition and a preparation method therefor and use thereof.

### BACKGROUND

Postsurgical pain is acute pain that occurs immediately after surgery. It usually lasts no more than 3-7 days, and may progress to chronic pain if not adequately controlled in the initial state. At present, the commonly used clinical treatment is the analgesic pump treatment. However, drugs contained in the analgesic pump are mainly opioid analgesics and some auxiliary analgesics such as tramadol and the like, which are effective, but come with a series of side effects such as respiratory depression, nausea and vomiting, hypotension, potential addiction and the like.

The adverse effects described above can be avoided by using local anesthetics to treat postsurgical pain. However, local anesthetics usually act for a short time. They last only several hours after being administered in a single dose, which are not enough for the treatment cycle for postsurgical pain. Therefore, the development of long-acting local anesthetic formulations is a current focus of research. The available long-acting local anesthetic formulations on the market include the bupivacaine multivesicular liposome injectable suspension developed by PACIRA under the trade name Exparel^{®}, which is used for treating postsurgical pain and nerve blocks and can produce analgesia for 24 h. A good sustained-release effect can be achieved by encapsulating bupivacaine in multivesicular liposomes. However, the process for preparing multivesicular liposomes is complicated and they need to be stored under strict conditions.

XARACOLL^{®} bupivacaine HCl implant of Innocoll was approved by the US Food and Drug Administration (FDA) on Aug. 28, 2020 for use in adult inguinal hernia repair to produce analgesia at the administration site for 24 h. However, the XARACOLL^{®} sponge is relatively great in volume and thus may cause a swelling at the incision site. In addition, due to the major wound required in surgery, there is little room for expansion of the range of indications for it in a late stage. Zynrelef^{®} (HTX-011) compound bupivacaine and meloxicam solution developed by Heron Therapeutics Inc was approved by the European Medicines Agency (EMA) on Sept. 24, 2020. It has become the third long-acting bupivacaine formulation in the world. Zynrelef uses a polymeric material polyorthoester as a sustained-release carrier, which may pose a risk of slow degradation at the administration site.

In addition, DURECT developed a long-acting bupivacaine formulation POSIMIR^{®} that uses a small-molecule ester as a sustained-release carrier; a related patent CN101035562 discloses a pharmaceutical composition comprising bupivacaine, sucrose acetoisobutyrate (SAIB) and benzyl alcohol. At present, POSIMIR^{®} has been subjected to phase III clinical trials, but the clinical results are not statistically different from those of bupivacaine solution. Dr. Gilbert J. Grant developed a large multivesicular vesicle (LMVV) of bupivacaine, which has the same structure as the Exparel liposome, but is different in particle size, preparation process and stability. There is a risk of leaking when it is stored at 4 °C. At present, it has been subjected to phase I clinical trials. Hefei Cosource Pharmaceuticals Inc. developed a bupivacaine pamoate cocrystal HYR-PB21-LA, which can achieve an extended release at the injection site as its solubility is only 0.076 mg/mL. However, it has the disadvantages of the preparation process being complicated and the like.

A patent CN111655236A discloses a controlled-release pharmaceutical composition, which comprises a biocompatible and bioerodible semi-solid gel comprising castor oil, a gelling agent, bupivacaine and optionally a corticosteroid, an analgesic or an anti-inflammatory agent, wherein a ratio of castor oil to glyceride is from 10:1 to 6:3 (w/w). Due to the unsaturated fatty chain contained in castor oil, there may be a risk of oxidation during storage. Moreover, due to the high proportion of castor oil, there may be a risk of oil separation as the storage is prolonged.

Therefore, there is a need to develop a sustained-release formulation system suitable for pharmaceutical use and having improved stability, safety, tolerability and/or sustained-release performance.

### SUMMARY

In order to solve the problems in the prior art, the present disclosure provides a pharmaceutical composition, which comprises the following components:
a. a liquid oil
b. a pharmaceutically acceptable gelator, selected from one or more of fatty acid glycerides of formula I: 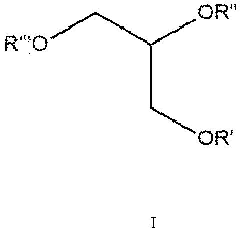 wherein R' and R" may be identical or different and are each independently selected from H or RaCO, and R‴ is selected from RaCO; each Ra is independently selected from saturated or unsaturated aliphatic hydrocarbyl;
c. a pharmaceutically acceptable stabilizer, selected from one or more of compounds of formula II or formula III: the compound of formula II being 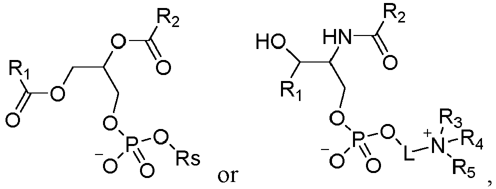 wherein Rs is selected from H, 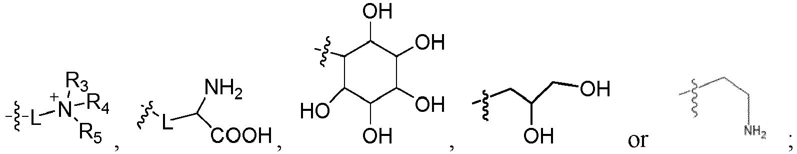 R₁ and R₂ are identical or different and are each independently selected from saturated or unsaturated aliphatic hydrocarbyl; R₃, R₄ and R₅ are identical or different and are each independently selected from H or alkyl; L is selected from alkylene; the compound of formula III being 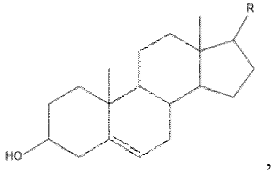 wherein R is selected from alkyl; and
d. at least one pharmaceutically active ingredient.

According to an embodiment of the present disclosure, in the formula I, each Ra is independently selected from alkyl; preferably, each Ra is independently selected from C₁₋₄₀ alkyl; more preferably, each Ra is independently selected from C₇₋₄₀ alkyl.

In some embodiments, R' and R" are selected from H, and R‴ is selected from (C₁₁₋₄₀ alkyl)C(=O); in some embodiments, R' is selected from H, R" is selected from RaCO, and a total number of carbon atoms in the Ra alkyl selected for each of R" and R‴ independently is greater than 18; in some embodiments, R', R" and R‴ are all selected from RaCO, and a total number of carbon atoms in the Ra alkyl selected for each of R', R" and R‴ independently is greater than 17.

According to an embodiment of the present disclosure, the component b pharmaceutically acceptable gelator includes one or more of glyceryl laurate, glyceryl palmitate, glyceryl myristate, glyceryl stearate, C₈-C₁₈ mixed fatty acid glycerides, glyceryl behenate, glyceryl palmitate stearate, glyceryl cocoate, hydrogenated coco-glyceride and hydrogenated palm glyceride.

Preferably, the component b pharmaceutically acceptable gelator includes, for example, one or more of glyceryl monostearate, glyceryl distearate, glyceryl tristearate, glyceryl monobehenate, glyceryl dibehenate, glyceryl monopalmitate stearate, glyceryl dipalmitate stearate, glyceryl monopalmitate, glyceryl dipalmitate and glyceryl palmitate stearate.

According to an embodiment of the present disclosure, in the formula II, R₁ and R₂ are identical or different and are each independently selected from C₁₀₋₃₀ saturated or unsaturated aliphatic hydrocarbyl, e.g., C₁₃₋₂₁ alkyl; R₃, R₄ and R₅ are identical or different and are each independently selected from H or C₁₋₁₀ alkyl, for example, from H, methyl or ethyl; L is selected from C₁₋₁₀ alkylene; preferably, L is selected from C₁₋₆ aklylene, and for example, is methylene or ethylidene. According to an embodiment of the present disclosure, in the compound of formula III, R is selected from C₁₋₁₀ alkyl, e.g., C₈₋₁₀ alkyl.

According to an embodiment of the present disclosure, the component c pharmaceutically acceptable stabilizer is selected from one or more of HSPC (hydrogenated soybean phosphatidylcholine), DMPC (dimyristoylphosphatidylcholine), DPPC (dipalmitoylphosphatidylcholine), DSPC (distearoylphosphatidylcholine), DLPC (dilauroylphosphatidylcholine), SPC soybean phosphatidylcholine (soybean phospholipid), EPC (egg yolk phospholipid), rapeseed phospholipid, sunflower phospholipid, DEPC (dierucoylphosphatidylcholine), DOPC (dioleoylphosphatidylcholine), POPC (palmitoyloleoylphosphatidylcholine), sphingomyelin, distearoyl phosphatidic acid (DSPA), dioleoylphosphatidylethanolamine (DOPE), dipalmitoyl phosphatidic acid (DPPA), myristoyl lysophosphatidylcholine (M-lysoPC), palmitoyl lysophosphatidylcholine (P-lysoPC), 1-stearoyl lysophosphatidylcholine (S-lysoPC), dipalmitoylphosphatidylethanolamine (DPPE), distearoylphosphatidylethanolamine (DSPE), dioleoylphosphatidylglycerol (DOPG), dimyristoylphosphatidylethanolamine (DMPE), dimyristoylphosphatidylglycerol (DMPG), dipalmitoylphosphatidylglycerol (DPPG), 1-palmitoyl-2-oleoylphosphatidylglycerol (POPG), distearoylphosphatidylglycerol (DSPG), dipalmitoylphosphatidylserine (DPPS), phosphatidylinositol (PI) and cholesterol (CHO).

According to an embodiment of the present disclosure, the pharmaceutical composition also comprises e. at least one pharmaceutically acceptable solvent.

According to an embodiment of the present disclosure, the pharmaceutical composition may further comprise g. a pharmaceutically acceptable release modifier.

According to an embodiment of the present disclosure, the pharmaceutical composition may further comprise f. a pharmaceutically acceptable acid.

According to an embodiment of the present disclosure, the liquid oil is selected from one or a combination of more of castor oil, sesame oil, corn oil, soybean oil, olive oil, safflower oil, cottonseed oil, peanut oil, fish oil, tea oil, almond oil, babassu oil, blackcurrant seed oil, borage oil, canola oil, palm oil, palm kernel oil, sunflower oil, medium-chain triglyceride, glyceryl dioleate and glyceryl monooleate.

According to an embodiment of the present disclosure, the at least one pharmaceutically active ingredient is not limited to a therapeutic type, and may be an anti-inflammatory drug, a local anesthetic, an analgesic, an anti-psychiatric drug, an anxiolytic, a sedative-hypnotic drug, an antidepressant, an antihypertensive drug, a steroid hormone, an antiepileptic drug, an antiseptic, an anticonvulsant, an anti-parkinson drug, a central nervous stimulant, an antipsychotic, an antiarrhythmic, an antianginal, an antithyroid drug, an antidote, an antiemetic, a hypoglycemic drug, an anti-tubercular drug, an anti-HIV drug, an anti-HBV drug, an antineoplastic, an anti-rejection drug or a mixture thereof.

According to an embodiment of the present disclosure, a suitable pharmaceutically active ingredient may be selected from one or a combination of more of the following compounds: aspirin, acetaminophen, benorilate, indomethacin, sulindac, diclofenac, diclofenac potassium, diclofenac sodium, ibuprofen, naproxen, flurbiprofen, flurbiprofen axetil, loxoprofen, nabumetone, ketorolac, phenylbutazone, bufexamac, fenoprofen, celecoxib, rofecoxib, polmacoxib, nimesulide, meloxicam, lornoxicam, piroxicam, etodolac, valdecoxib, parecoxib, imrecoxib, lumiracoxib, bupivacaine, levobupivacaine, ropivacaine, mepivacaine, lidocaine, procaine, benzocaine, tetracaine, dyclonine, enkephalin, dynorphin, β-endorphin, naltrexone, buprenorphine, morphine, dimethylmorphine, codeine, dihydrocodeine, oxycodone, hydrocodone, nalbuphine, fentanyl, sufentanil, remifentanil, tramadol, desmethyltramadol, tapentadol, dezocine, pentazocine, methadone, meperidine, ketamine, diazepam, lormetazepam, lisdexamfetamine, dextropropoxyphene, difelikefalin, oliceridine, chlorpromazine, triflupromazine, mesoridazine, piperacetazine, thioridazine, chlorprothixene, diazepam, alprazolam, clonazepam, oxazepam, imipramine, amitriptyline, doxepin, nortriptyline, amoxapine, tranylcypromine, phenelzine, procainamide, isoamyl nitrite, nitroglycerin, propranolol, metoprolol, prazosin, phentolamine, trimethaphan, captopril, enalapril, clonidine, dexmedetomidine, adrenaline, noradrenaline, tizanidine, α-methyldopa, glycopyrrolate, cortisone, hydrocortisone, betamethasone, triamcinolone acetonide, dexamethasone, dexamethasone ester, prednisone, prednisolone, methylprednisolone, beclomethasone, clobetasol, progesterone, testosterone, testosterone enanthate, testosterone undecanoate, testosterone cypionate, progesterone, fulvestrant, allopregnanolone, ganaxolone, phenytoin, ethotoin, benzalkonium chloride, benzethonium chloride, mafenide acetate, methylbenzethonium chloride, nitrofural, nitromersol, phenobarbital, amobarbital, pentobarbital, secobarbital, carbidopa, levodopa, aniracetam, oxiracetam, piracetam, doxapram, aripiprazole, olanzapine, haloperidol, quetiapine, risperidone, clozapine, paliperidone, atenolol, bisoprolol, metoprolol, atenolol, amlodipine, nimodipine, isosorbide mononitrate, epoprostenol, treprostinil, iloprost, beraprost, methimazole, propylthiouracil, propranolol, naloxone, lofexidine, flumazenil, amphetamine, granisetron, ondansetron, tropisetron, dolasetron, palonosetron, scopolamine, domperidone, glipizide, glyburide, glimepiride, glibenclamide, gliclazide, tolbutamide, liraglutide, exenatide, dulaglutide, sermaglutide, darunavir, dolutegravir sodium, emtricitabine, raltegravir, ritonavir, stavudine, nevirapine, zidovudine, stavudine, etravirine, adefovir dipivoxil, entecavir, telbivudine, lamivudine, tenofovir dipivoxil, tenofovir alafenamide, thiosemicarbazide, pyrazinamide, prothionamide, cyclophosphamide, 5-fluorouracil, carmustine, lomustine, melphalan, chlorambucil, methotrexate, vincristine, bleomycin, doxorubicin, tamoxifen, cyclosporine, tacrolimus, everolimus, sirolimus, and pharmaceutically acceptable salts, stereoisomers and derivatives of the compounds.

According to an embodiment of the present disclosure, the pharmaceutically active ingredient is selected from amide local anesthetics, for example, from bupivacaine, ropivacaine, levobupivacaine, mepivacaine, lidocaine and salts thereof. The salts of the amide local anesthetics may be selected from fatty acid salts and water-soluble salts thereof, and acids for forming the salts include lauric acid, myristic acid, stearic acid, palmitic acid, behenic acid, arachidic acid, hydrochloric acid, sulfonic acid, phosphoric acid, acetic acid, citric acid, maleic acid, methanesulfonic acid, fumaric acid, succinic acid, lactic acid and the like.

According to an embodiment of the present disclosure, the pharmaceutically active ingredient may further comprise a second active ingredient in addition to the amide local anesthetic, and the pharmaceutically active ingredient may be selected from one of a COX receptor inhibitor, an adrenoceptor agonist and a glucocorticoid drug. The COX receptor inhibitor includes non-selective COX inhibitors and selective COX-2 inhibitors. In these categories, representative non-steroidal anti-inflammatory drugs include, but are not limited to, the following non-selective COX inhibitors: aspirin, acetaminophen, benorilate, indomethacin, sulindac, diclofenac, diclofenac potassium, diclofenac sodium, ibuprofen, naproxen, flurbiprofen, loxoprofen, nabumetone, piroxicam, ketorolac, phenylbutazone, bufexamac and fenoprofen; and the following selective COX-2 inhibitors: celecoxib, rofecoxib, nimesulide, meloxicam, lornoxicam, etodolac, valdecoxib, parecoxib, imrecoxib and lumiracoxib; and pharmaceutically acceptable salts, stereoisomers and derivatives of the compounds. The adrenoceptor agonist is mainly an α2-adrenoceptor agonist, including but not limited to, clonidine, dexmedetomidine, adrenaline, noradrenaline, tizanidine and α-methyldopa. The glucocorticoid drug includes, but are not limited to, cortisone, hydrocortisone, betamethasone, triamcinolone acetonide, dexamethasone, prednisone, prednisolone, methylprednisolone, beclomethasone and clobetasol.

In some embodiments, the pharmaceutically active ingredient is selected from one or a combination of more of ropivacaine, bupivacaine, levobupivacaine, meloxicam, celecoxib, ketorolac and triamcinolone acetonide. In some embodiments, the pharmaceutically active ingredient is selected from a combination of an amide local anesthetic and a non-steroidal anti-inflammatory drug, e.g., a composition of ropivacaine and meloxicam, a composition of levobupivacaine and meloxicam, a composition of bupivacaine and meloxicam, a composition of ropivacaine and celecoxib, a composition of levobupivacaine and celecoxib, a composition of bupivacaine and celecoxib, and the like.

According to an embodiment of the present disclosure, the pharmaceutically acceptable release modifier is selected from small-molecule esters and surfactants. The small-molecule esters are glyceryl triacetate, isopropyl stearate, isopropyl laurate, isopropyl palmitate, isopropyl myristate and benzyl benzoate.

In some embodiments, the surfactants are nonionic surfactants.

In some embodiments, the surfactants include polyoxyl 40 stearate, caprylic capric polyethylene glycol glyceride, lauroyl polyoxyethylene glyceride, stearoyl polyoxyethylene glyceride, oleoyl polyoxyethylene glyceride, vitamin E polyethylene glycol succinate, egg yolk phosphatidylcholine, soybean phospholipid, hydrogenated soybean phospholipid, poloxamer, polysorbate, polyethylene glycol-12-hydroxystearate, propylene glycol monocaprylate, etc. The poloxamer may be selected from, for example, poloxamer 407 and poloxamer 188; the polysorbate may be selected from, for example, polysorbate 80.

According to an embodiment of the present disclosure, the pharmaceutically acceptable acid (component f) is selected from acetic acid, lactic acid, succinic acid, fumaric acid, maleic acid, methanesulfonic acid, linoleic acid, sorbic acid, caprylic acid, pelargonic acid, lauric acid, palmitic acid, oleic acid, hydrochloric acid, phthalic acid, capric acid, myristic acid, propionic acid, butyric acid, heptanoic acid, valeric acid, malic acid, tartaric acid, oxalic acid, citric acid, ascorbic acid, salicylic acid, caffeic acid, vitamin E succinic acid, and the like.

According to an embodiment of the present disclosure, the composition may further comprise one or more antioxidants. The antioxidants can be used to prevent or reduce oxidation of the phospholipid or liquid oil in the sustained-release drug delivery system described in the present disclosure. The antioxidants provided herein include, but are not limited to, vitamin C (ascorbic acid), cysteine or hydrochloride thereof, vitamin E (tocopherol), ascorbyl palmitate, glutathione, alpha lipoic acid, thioglycerol, butyl hydroxy anisole, butylated hydroxytoluene, ethylenediaminetetraacetic acid and sodium salts thereof, citric acid and tartaric acid.

According to an embodiment of the present disclosure, the composition may further comprise other conventional excipients in the pharmaceutical art. Examples of suitable pharmaceutically acceptable excipients are described in Excipients and their use in injectable products. PDA J Pharm Sci Technol., Jul-Aug 1997, 51:166-171; and Excipient Selection In Parenteral Formulation Development, Pharma Times, Mar 2013, 45(3):65-77, which are incorporated herein by reference in their entirety. According to an embodiment of the present disclosure, the liquid oil accounts for about 20% to about 90% (w/w), for example, about 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78% or 79%, of a total amount of the composition. In some embodiments, a proportion of the liquid oil is about 30% to 90% (w/w). In some embodiments, a proportion of the liquid oil is about 40% to 79% (w/w).

According to an embodiment of the present disclosure, the gelator accounts for 2% to 50% (w/w), for example, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% or 50%, of the total amount of the composition. In some embodiments, the gelator accounts for 2% to 30% (w/w) of the total amount of the composition.

According to an embodiment of the present disclosure, the stabilizer accounts for 1% to 40% (w/w), for example, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39% or 40%, of the total amount of the composition. In some embodiments, the stabilizer accounts for 5% to 30% (w/w) of the total amount of the composition.

According to an embodiment of the present disclosure, the pharmaceutically active ingredient accounts for 0.01% to 50.0% (w/w) of the total amount of the composition. According to an embodiment of the present disclosure, the pharmaceutically active ingredient accounts for 0.01% to 15% (w/w), for example, 0.01%, 0.05%, 0.1%, 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5% or 15.0%, of the total amount of the composition. According to some embodiments, the pharmaceutically active ingredient is present in an amount of 3% (w/w) to 10% (w/w). According to some embodiments, when the pharmaceutically active ingredient is selected from two or more, each pharmaceutically active ingredient may account for 0.01% to 10% (w/w), for example, 0.01%, 0.05%, 0.1%, 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5% or 10.0%, of the total amount of the composition.

According to an embodiment of the present disclosure, the total amount of the solvent accounts for 0% to 50% (w/w) of the total amount of the composition; in some embodiments, the composition may comprise no solvent; in some embodiments, the total amount of the solvent may account for 0.01% to 50%, preferably 2% to 50%, for example, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% or 50%, of the total amount of the composition. In some embodiments, the total amount of the solvent accounts for 5% to 50% (w/w) of the total amount of the composition; in some embodiments, the total amount of the solvent accounts for 5% to 30% (w/w) of the total amount of the composition. When the solvent is selected from a combination of two or more, each solvent may account for 0% to 30% (w/w), preferably 0.01% to 30%, for example, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 11%, 12%, 13%, 14%, 15%, 16%, 17%, 18%, 19%, 20%, 21%, 22%, 23%, 24%, 25%, 26%, 27%, 28%, 29% or 30%, of the total amount of the composition.

According to an embodiment of the present disclosure, the solvent is a non-aqueous solvent selected from one or a combination of more of an alcohol, *N*-methyl pyrrolidone, benzyl benzoate and dimethylsulfoxide. The alcohol is selected from methanol, ethanol, n-propanol, isopropanol, n-butanol, isobutanol, tert-butanol, ethylene glycol, propylene glycol, glycerol, benzyl alcohol, phenethyl alcohol and polyethylene glycol.

Preferably, the non-aqueous solvent is selected from one or a combination of more of benzyl alcohol, *N*-methyl pyrrolidone, dimethyl sulfoxide and absolute ethanol.

According to an embodiment of the present disclosure, the release modifier accounts for 0% to 40% (w/w), preferably 0.1% to 40% (w/w), of the total amount of the composition; in some embodiments, the release modifier may account for 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39% or 40% of the total amount of the composition; in some embodiments, when the release modifier is selected from a small-molecule ester, an amount of the release modifier is 1% to 35%; in some embodiments, when the release modifier is selected from a surfactant, an amount of the release modifier is 0.1% to 5% (w/w).

According to an embodiment of the present disclosure, the pharmaceutically acceptable acid (component f) accounts for 0% to 20% (w/w) of the total amount of the composition; in some embodiments, the composition may contain no acid; in some embodiments; the acid accounts for 0.01% to 20%, preferably 0.05% to 20%, for example, 0.05%, 0.1%, 0.5%, 1%, 2%, 3%, 4%, 5%, 6%, 7%, 8%, 9%, 10%, 11%, 12%, 13%, 14%, 15%, 16%, 17%, 18%, 19% or 20%, of the total amount of the composition; in some embodiments, the acid accounts for 4% to 20% of the total amount of the composition.

The pharmaceutical composition provided herein is a semi-solid formulation. In the pharmaceutical composition of the present disclosure, the pharmaceutically acceptable solvent and the release modifier may serve as a viscosity modifier so that the composition is suitable for injection. In some embodiments, a viscosity of the composition is less than 20,000 cP at 30 °C. In some embodiments, a viscosity of the composition is in the range of 5000 cP to 10,000 cP at 30 °C. In some embodiments, a viscosity of the composition is in the range of 3000 cP to 5000 cP at 30 °C. In some embodiments, a viscosity of the composition is in the range of 1000 cP to 3000 cP at 30 °C.

The present disclosure provides a preparation method for the pharmaceutical composition, which comprises the following steps:
(a1) mixing a liquid oil, a pharmaceutically acceptable gelator, a pharmaceutical stabilizer and a solvent, and stirring under a heating condition to obtain a clear and uniform mixed solution;
(a2) adding at least one pharmaceutically active ingredient to the mixed solution, and stirring to form a uniform mixture; and
(a3) cooling the uniform mixture formed in (a2) to room temperature.

According to an embodiment of the present disclosure, the mixing in step (a1) further comprises adding at least one release modifier; for example, step (a1) may be mixing a liquid oil, a gelator, a pharmaceutical stabilizer and a pharmaceutically acceptable solvent with a release modifier.

In some embodiments, the method comprises:
1. mixing a liquid oil, a gelator, a stabilizer, a pharmaceutically active molecule and a pharmaceutical solvent at 50-70 °C until a clear and transparent solution is obtained;
2. sterilizing the hot solution through a 0.22 µm membrane filter; and
3. cooling the filtered mixed solution to room temperature.

In some embodiments, the method comprises:
1. mixing a liquid oil, a gelator, a pharmaceutical stabilizer, a pharmaceutically active molecule, a pharmaceutical solvent and a release modifier at 50-70 °C until a clear and transparent solution is obtained;
2. sterilizing the hot solution through a 0.22 µm membrane filter; and
3. cooling the filtered mixed solution to room temperature.

In some embodiments, a less soluble active molecule is dissolved in part of the solvent first according to the natures of different pharmaceutically active molecules, and then added to a solution formed by heating and mixing of the liquid oil, the gelator and the remaining solvent to prepare the desired pharmaceutical composition.

In some embodiments, the method comprises:
1. mixing a liquid oil, a gelator and a stabilizer with part of a solvent at 50-70 °C until a clear and transparent solution is obtained;
2. adding a pharmaceutically active molecule to part of the solvent until it is completely dissolved;
3. mixing well the drug solution with the solution in step 1;
4. sterilizing the hot solution through a 0.22 µm membrane filter; and
5. cooling the filtered mixed solution to room temperature.

In some embodiments, a less soluble active molecule is dissolved in part of the solvent first according to the natures of different pharmaceutically active molecules, and then added to a solution formed by heating and mixing of the liquid oil, the gelator, part of the solvent and a release modifier to prepare the desired pharmaceutical composition.

In some embodiments, the method comprises:
1. mixing a liquid oil, a gelator, a stabilizer, part of a solvent and a release modifier at 50-70 °C until a clear and transparent solution is obtained;
2. adding a pharmaceutically active molecule to part of the solvent until it is completely dissolved;
3. mixing well the drug solution with the solution in step 1;
4. sterilizing the hot solution through a 0.22 µm membrane filter; and
5. cooling the filtered mixed solution to room temperature.

For the preparation method for the pharmaceutical composition of the present disclosure, in some embodiments, the mixed solution is naturally cooled to room temperature; in some embodiments, the mixed solution is rapidly cooled to be solidified and then left at room temperature; in some embodiments, the mixed solution is rapidly cooled to be solidified, then incubated at a particular temperature for a certain period of time and then left at room temperature; in some embodiments, the mixed solution is incubated at the melting temperature of the system to be solidified and then left at room temperature; in some embodiments, the mixed solution is incubated at the melting temperature of the system to be solidified, then incubated at a particular temperature for a certain period of time and then left at room temperature.

The present disclosure provides a sustained-release formulation comprising the pharmaceutical composition. The formulation is administered as a depot formulation. In one aspect, the formulation is injectable. In another aspect, the formulation may be administered topically.

In another aspect, the formulation may be injected subcutaneously, injected perineurally, injected intramuscularly, or administered directly to a wound.

In another aspect, the formulation is suitable for being administered to a skin or mucosa.

The formulation provided herein is administered in a single dose, and the amount of the drug contained can produce analgesia and nerve blocks and can be used to prevent or relieve local pain. According to some embodiments, the formulation provided herein can form a depot in a stable form at the administration site to slowly and continuously release the drug, so that the release of local anesthetic is extended and thus the therapeutic effect is improved.

In some embodiments, the formulation can effectively produce a therapeutic effect for at least 24 h after being administered. In some embodiments, the formulation can effectively produce a therapeutic effect for at least 24 h to 48 h after being administered. In some embodiments, the formulation can effectively produce a therapeutic effect for at least 48 h to 72 h after being administered. In some embodiments, the formulation can effectively produce a therapeutic effect for at least 72 h after being administered. According to an embodiment of the present disclosure, the formulation further comprises packaging filled with the formulation, the packaging being selected from one or more of a vial, a pre-filled syringe and a cartridge.

### Terminology and Abbreviations

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the present specification.

When a numerical range defined by "integers" or "integers" only is included in the specification and claims of this application, it shall be construed as including both endpoints of the range and every integer within the range. For example, "an integer of 0-10" shall be construed to include each of integers 0, 1, 2, 3, 4, 5, 6, 7, 8, 9 and 10.

When the numerical range is defined by "numbers" or may include "integers" or "non-integers", it shall be construed as including both endpoints of the range, every integer within the range, and every decimal within the range. For example, "numbers of 0-10" shall be construed as including not only each of integers 0, 1, 2, 3, 4, 5, 6, 7, 8, 9 and 10, but also at least the sums of each integer and 0.1, 0.2, 0.3, 0.4, 0.5, 0.6, 0.7, 0.8 or 0.9.

The term "aliphatic hydrocarbyl" includes saturated or unsaturated, linear or branched chain hydrocarbon groups. The aliphatic hydrocarbyl may be selected from alkyl (saturated aliphatic hydrocarbyl), alkenyl, alkynyl and the like. The number of carbon atoms in the aliphatic hydrocarbyl is preferably 1-40, more preferably 1-30 (e.g., C1, C2, C3, C4, C5, C6, C7, C8, C9, C10, C11, C12, C13, C14, C15, C16, C17, C18, C19, C20, C21, C22, C23, C24, C25, C26, C27, C28, C29, C30, C31, C32, C33, C34, C35, C36, C37, C38, C39 or C40). Specifically, the aliphatic hydrocarbyl includes, but is not limited to: methyl, ethyl, n-propyl, isopropyl, n-butyl, isobutyl, tert-butyl, *n-*pentyl, isopentyl, neopentyl, n-hexyl, ethenyl, 1-propenyl, 2-propenyl, 1-methylethenyl, 1-butenyl, 1-ethylethenyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl and 1-hexynyl. The "aliphatic hydrocarbyl" moieties contained in other groups are as described above.

The term "alkyl" refers to a saturated aliphatic hydrocarbon group, which complies with the related definition for aliphatic hydrocarbyl described above. For example, the number of carbon atoms in the alkyl is preferably 1-40, more preferably 1-30, or 1-10 (e.g., C1, C2, C3, C4, C5, C6, C7, C8, C9, C10, C11, C12, C13, C14, C15, C16, C17, C18, C19, C20, C21, C22, C23, C24, C25, C26, C27, C28, C29, C30, C31, C32, C33, C34, C35, C36, C37, C38, C39 or C40). Those skilled in the art can understand that reference can be made, for "alkylene", to the related definitions for aliphatic hydrocarbyl described above. For example, the number of carbon atoms in the alkylene may be C1, C2, C3, C4, C5, C6, C6, C7, C8, C9 or C10.

The term "biocompatibility" refers to the interaction between the components of a composition and an organism.

The term "active ingredient" refers to a drug for treating a disease. Therefore, the terms active ingredient and drug are used interchangeably. As used herein, the term "active ingredient" or "drug" includes, but is not limited to, pharmaceutically active substances that act topically or systemically, and it can be administered topically or by injection, such as subcutaneous, intradermal, intramuscular and intra-articular injection. At least one active ingredient is present in the sustained-release drug delivery system of the present disclosure.

The term "oleogel" refers to a thermally reversible, semi-solid dispersion system with certain viscoelasticity formed by adding a gelator to a liquid oil.

The term "gelator" refers to a class of substances that have lipophilic structures and interactable sites in the molecule and have certain surface activity and thermal reversibility.

The term "semi-solid" refers to a form that can flow at a certain pressure. More specifically, the semi-solid typically has a viscosity between 100 cP and 50,000 cP at 30 °C, particularly between 100 cP and 20,000 cP at 30 °C.

The term "small-molecule ester" refers to an ester with a molecular weight of less than 500, which is liquid at room temperature.

As used herein, the term "amide" refers to an amide or caine local anesthetic, e.g., bupivacaine, levobupivacaine, ropivacaine, mepivacaine, lidocaine, etc. An amide local anesthetic generally consists of a lipophilic moiety and a hydrophilic moiety. The lipophilic moiety may be an aromatic hydrocarbon or aromatic heterocyclic ring, and the benzene ring has the best effect. The activity can be enhanced by introducing electron-donating groups such as amino groups and the like into the benzene ring. The hydrophilic moiety is typically a secondary amine, a tertiary amine or pyrrolidine, piperidine, morpholine, etc., and is most commonly a tertiary amine. The pKa is typically between 7.5 and 7.9, and it is ionic under physiological conditions.

The abbreviations used in the present disclosure are defined as follows: SPC for soybean phosphatidylcholine (soybean phospholipid), EPC for egg yolk phosphatidylcholine (egg yolk phospholipid), HSPC for hydrogenated soybean phosphatidylcholine, DLPC for dilauroylphosphatidylcholine, DMPC for dimyristoylphosphatidylcholine, DPPC for dipalmitoylphosphatidylcholine, DSPC for distearoylphosphatidylcholine, DEPC for dierucoylphosphatidylcholine, DOPC for dioleoylphosphatidylcholine, POPC for palmitoyloleoylphosphatidylcholine, DSPA for distearoyl phosphatidic acid, DOPE for dioleoylphosphatidylethanolamine, DPPA for dipalmitoyl phosphatidic acid, M-lysoPC for myristoyl lysophosphatidylcholine, P-lysoPC for palmitoyl lysophosphatidylcholine, S-lysoPC for 1-stearoyl lysophosphatidylcholine, DPPE for dipalmitoylphosphatidylethanolamine, DSPE for distearoylphosphatidylethanolamine, DOPG for dioleoylphosphatidylglycerol, DMPE for dimyristoylphosphatidylethanolamine, DMPG for dimyristoylphosphatidylglycerol, DPPG for dipalmitoylphosphatidylglycerol, POPG for 1-palmitoyl-2-oleoylphosphatidylglycerol, DSPG for distearoylphosphatidylglycerol, DPPS for dipalmitoylphosphatidylserine, PI for phosphatidylinositol, BA for benzyl alcohol, NMP for N-methylpyrrolidone, DMSO for dimethyl sulfoxide, BHA for butyl hydroxy anisole, BHT for butylated hydroxytoluene, BUP for bupivacaine, ROP for ropivacaine, MLX for meloxicam, CHO for cholesterol, PRX for piroxicam, LBUP for levobupivacaine, TAC for triamcinolone acetonide, KTL for ketorolac, VE for vitamin E, and VES for vitamin E succinic acid.

### Beneficial Effects

1) The present disclosure provides a sustained-release formulation composition, which is semi-solid at room temperature and can be directly used as a drug depot at the administration site. The gelator selected herein is well biocompatible with other components of the formulation, and enables the liquid oil to be solidified to meet the requirement of sustained release of the drug.
2) The present disclosure surprisingly reveals that by adding a suitable stabilizer to the formulation system, the discoloration problem presented during long-term storage can be reduced, the oil-holding capacity of the composition can be improved, the gel property of the composition can be enhanced, and the viscosity of the composition can be reduced, and the like.
3) The sustained-release drug delivery liquid composition of the present disclosure has a suitable viscosity, which favors administration; the sustained-release drug delivery semi-solid composition of the present disclosure can be directly administered to the administration site, which favors clinical administration.
4) The composition of the present disclosure causes less irritation and shows good medication safety and tolerability.
5) The sustained-release formulation system provided herein can achieve good release performance of the pharmaceutically active ingredient and reduce the possibility of burst release.
6) The composition of the present disclosure is particularly suitable for the development of pharmaceutical formulations with anesthetic and analgesic activities and has more advantages over other sustained-release analgesic systems; for example, the release of the analgesic active ingredient is lasting and stable; it can be administered by injection, and is also suitable for stable and convenient topical administration; it is well tolerable in patients and has less side effects.

### BRIEF DESCRIPTION OF THE DRAWINGS

FIGs. 1-1 and 1-2 show rheological results of composition 1010 at 2-8 °C and 25 °C.
FIGs. 1-3 and 1-4 show rheological results of composition 1013 at 2-8 °C and 25 °C.

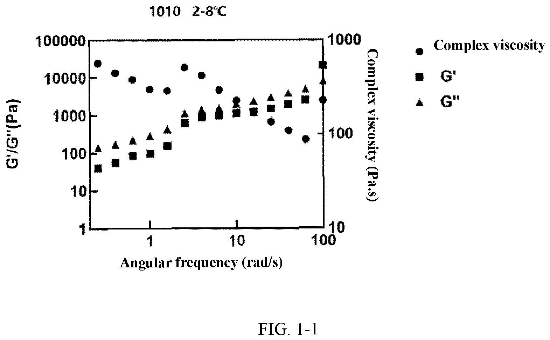

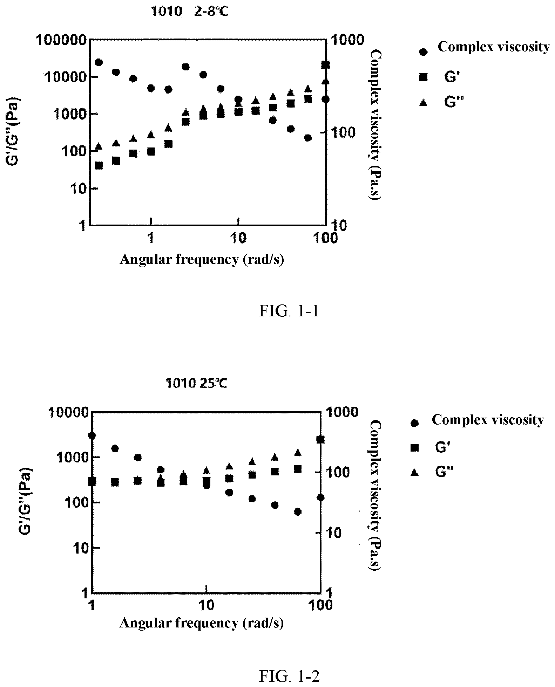

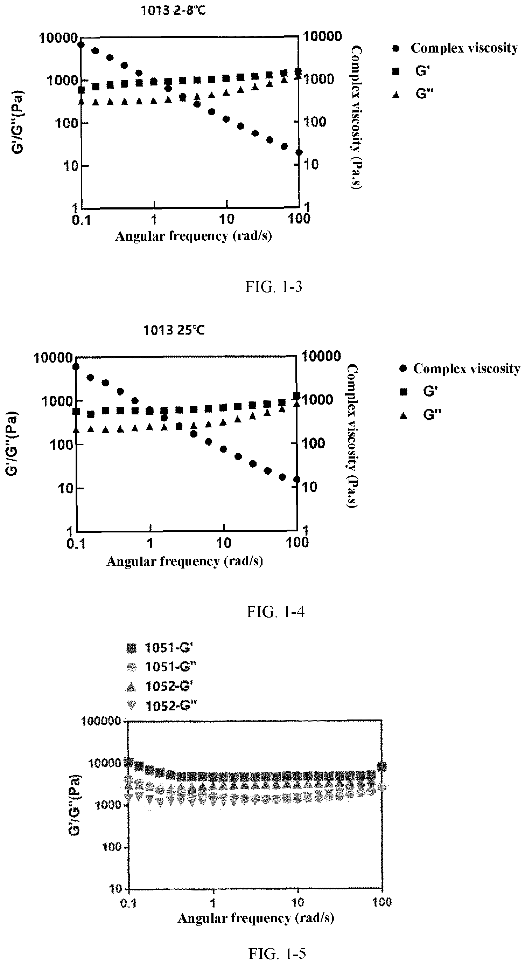

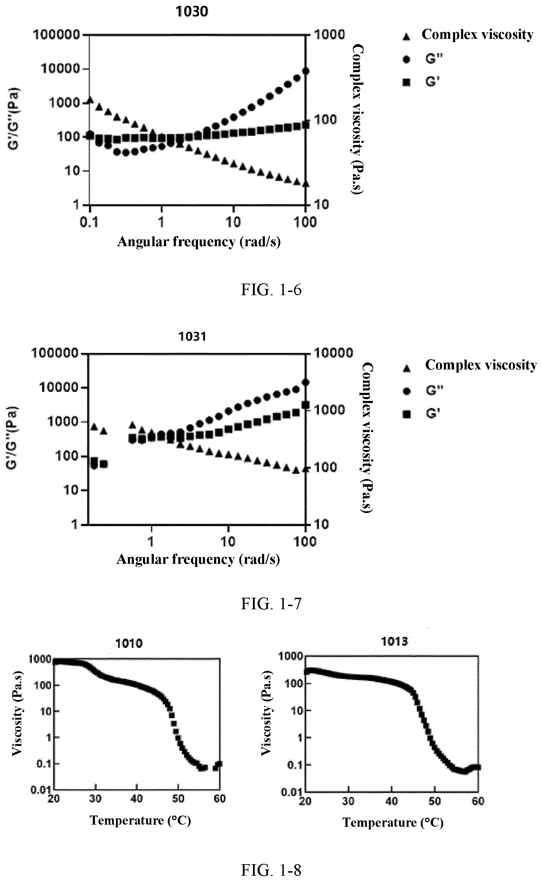

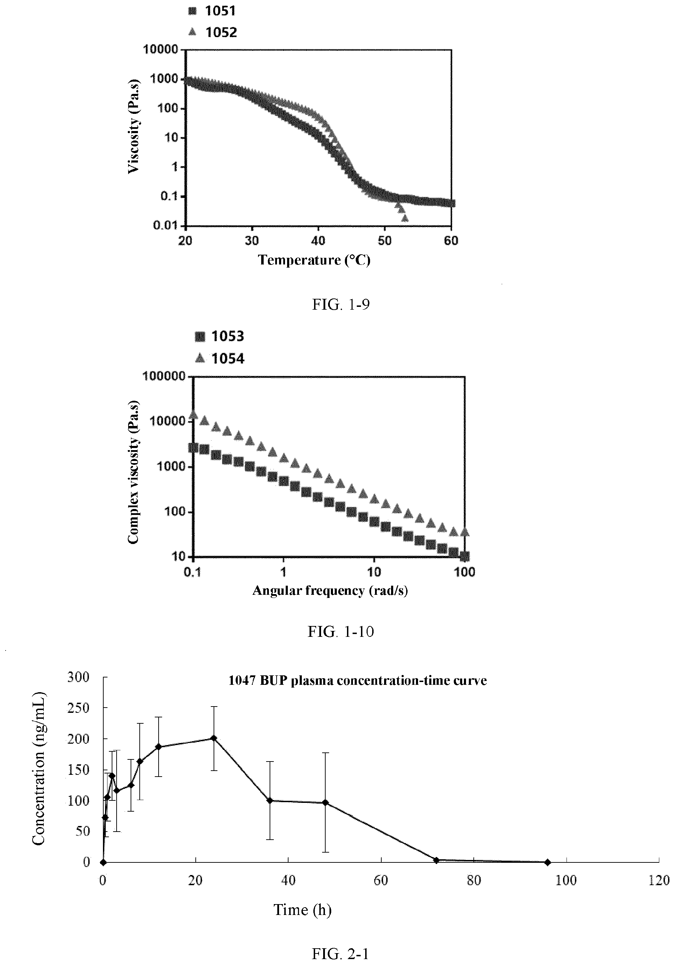

FIGs. 1-5 to 1-7 show rheological results of compositions 1051, 1052, 1030 and 1031.
FIG. 1-8 shows graphs of viscosity-temperature changes for compositions 1010 and 1013.
FIG. 1-9 shows graphs of viscosity-temperature changes for compositions 1051 and 1052.
FIG. 1-10 shows graphs of viscosity-rotational speed changes for compositions 1053 and 1054.

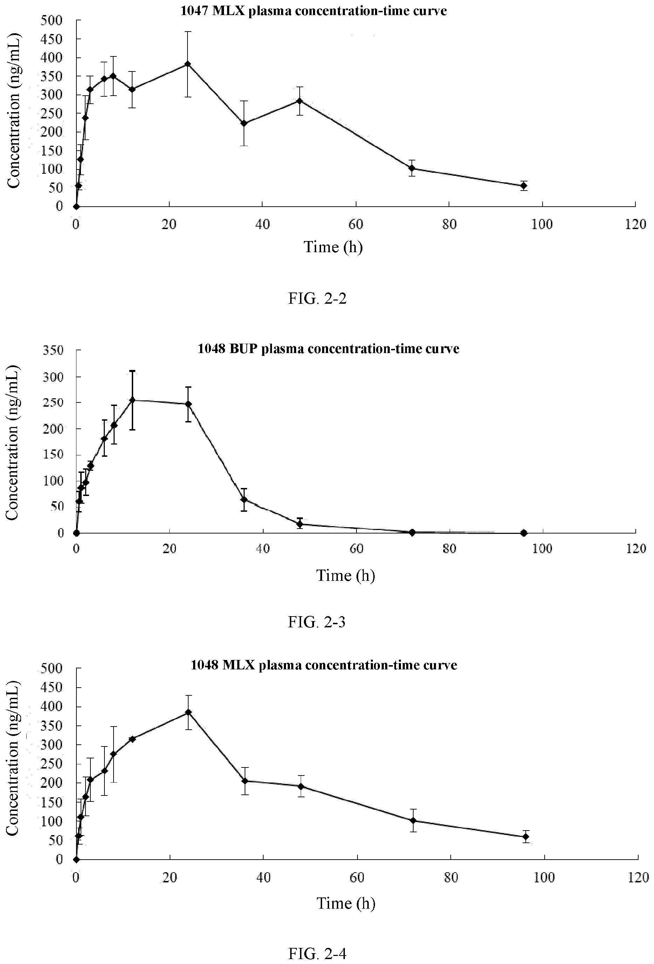

FIGs. 2-1 and 2-2 show bupivacaine and meloxicam plasma concentration-time curves for composition 1047.
FIGs. 2-3 and 2-4 show bupivacaine and meloxicam plasma concentration-time curves for composition 1048.

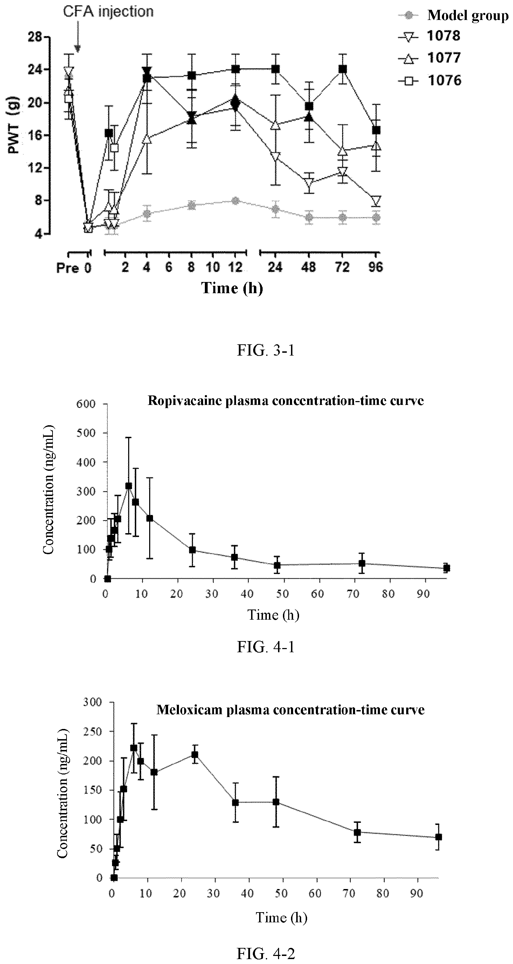

FIG. 3-1 shows the effects of compositions 1076, 1077 and 1078 on the mechanical hyperalgesia of a rat CFA inflammatory pain model. Pre indicates the basal mechanical pain threshold before molding, and the black solid dots indicate statistical differences (p<0.05) in the results relative to the model group.
FIGs. 4-1 and 4-2 show ropivacaine and meloxicam plasma concentration-time curves for composition 1079.

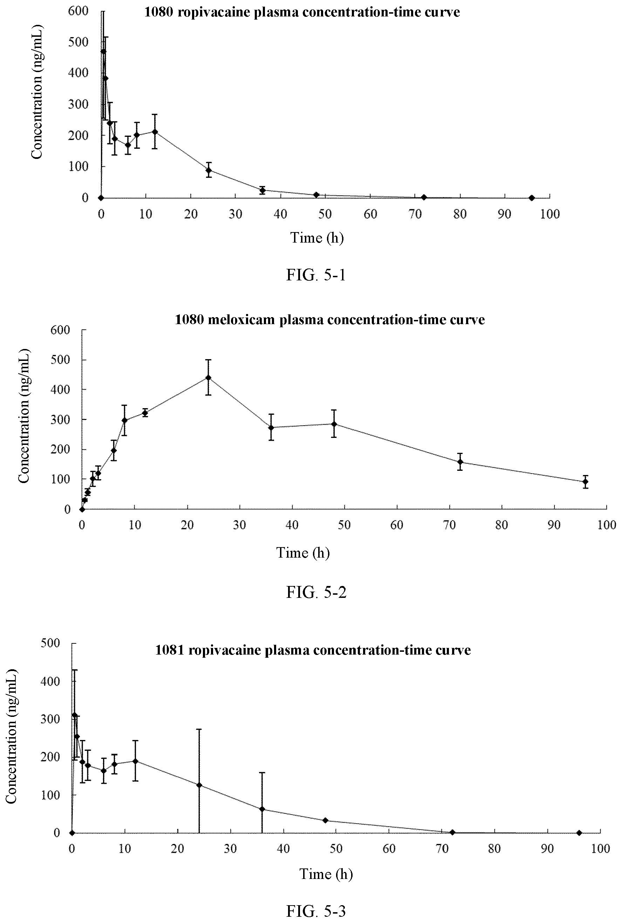

FIGs. 5-1 and 5-2 show ropivacaine and meloxicam plasma concentration-time curves for composition 1080.

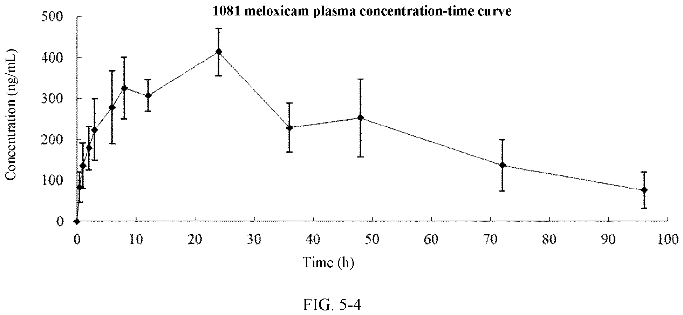

FIGs. 5-3 and 5-4 show ropivacaine and meloxicam plasma concentration-time curves for composition 1081.

### DETAILED DESCRIPTION

The technical scheme of the present disclosure will be further illustrated in detail with reference to the following specific examples. It should be understood that the following examples are merely exemplary illustration and explanation of the present disclosure, and should not be construed as limiting the protection scope of the present disclosure. All techniques implemented based on the content of the present disclosure described above are encompassed within the protection scope of the present disclosure.

Unless otherwise stated, the starting materials and reagents used in the following examples are all commercially available products or can be prepared using known methods.

### Materials:

Castor oil: CO;
Glyceryl monostearate: IMWITOR^{®} 900K; Cithrol GMS 40, Geleol;
Mixed fatty acid glycerides (stearin): stearin 36, stearin 38, SUPPOCIRE AM, SUPPOCIRE CM, GELUCIRE 43/01, SOFTISAN 378;
Glyceryl distearate: Precirol ATO5;
Glyceryl behenate: COMPRITOL 888 ATO;
Phospholipid: SPC, HSPC, DMPC, DPPC, DSPC, DEPC, EPC, and DOPC.

### Example 1

### Effects of Stabilizers on Appearance of Compositions

Compositions comprising different gelators were prepared according to Tables 1-1 to 1-3, and let stand at room temperature for a long period of 20 months, and the compositions were compared with standard colorimetric solutions Y (yellow) 1-10 to examine the compositions for changes in apparent color.

**Table 1-1. Apparent colors of compositions comprising glyceryl monostearate as gelator after standing for a long period of time**

| No. | CO | IMWITOR 900K | ROP | MLX | NMP | SPC | HSPC | BA | Corresponding color level | |
|---|---|---|---|---|---|---|---|---|---|---|
| | wt% | wt% | wt% | wt% | wt% | wt% | wt% | wt% | 0h | Month 20 |
| 1001 | 68.8 | 15 | 2.5 | 0.19 | 3.51 | 0 | 0 | 10 | Y6~7 | Y8-9 |
| 1002 | 75.3 | 8.5 | 2.5 | 0.19 | 3.51 | 0 | 0 | 10 | Y6~7 | Y>10 |
| 1003 | 59.1 | 22.2 | 5 | 0.19 | 3.51 | 0 | 0 | 10 | Y6~7 | Y8-9 |
| 1004 | 51.6 | 22.2 | 2.5 | 0.19 | 3.51 | 10 | 0 | 10 | Y6~7 | Y7~8 |
| 1005 | 58.8 | 22.2 | 2.5 | 0.19 | 3.51 | 0 | 10 | 10 | Y6~7 | Y7-8 |

**Table 1-2. Apparent colors of compositions comprising stearin as gelator after standing for a long period of time**

| No. | CO | Stearin 36 | Stearin 38 | ROP | MLX | NMP | BA | Corresponding color level | |
|---|---|---|---|---|---|---|---|---|---|
| | wt% | wt% | wt% | wt% | wt% | wt% | wt% | 0h | Month 20 |
| 1006 | 53.8 | 30 | 0 | 2.5 | 0.19 | 3.51 | 10 | Y6~7 | Y>10 |
| 1007 | 53.8 | 0 | 30 | 2.5 | 0.19 | 3.51 | 10 | Y6~7 | Y9-10 |

**Table 1-3. Apparent color of composition comprising glyceryl behenate as gelator after standing for a long period of time**

| No. | CO | COMPRITOL 888 ATO | ROP | MLX | NMP | BA | Corresponding color level | |
|---|---|---|---|---|---|---|---|---|
| | wt% | wt% | wt% | wt% | wt% | wt% | 0h | Month 20 |
| 1008 | 61.6 | 22.2 | 2.5 | 0.19 | 3.51 | 10 | Y6~7 | Y9-10 |

The present disclosure unexpectedly revealed that all the oleogel formulations comprising only a fatty acid glyceride as the gelator became darkened to varying degrees during long-term retention. After the compositions with added stabilizers (e.g., SPC and HSPC) were let stand for a long period of 20 months, their apparent color levels were between Y7 and Y8. Their color changes were significantly smaller than those of other oleogel compositions, indicating that the addition of stabilizers could significantly increase the stability of oleogel compositions in appearance.

### Example 2

### Study on Oil-Holding Capacity of Compositions

Pharmaceutical compositions comprising different proportions of the gelator were prepared according to each of the components shown in Tables 2-1 to 2-9 below. After 0.5 g of a pharmaceutical composition was weighed into a centrifuge tube and centrifuged at different rotational speeds, the centrifuge tube was inverted, and oil separation in the composition was observed.

**Table 2-1. Oil-holding capacity study of oleogel compositions comprising no stabilizer**

| No. | CO | SUPPOCIRE AM | SUPPOCIRE CM | BUP | GELUCIRE 43/01 | SOFTISAN 378 | 14000rpm10min |
|---|---|---|---|---|---|---|---|
| | wt% | wt% | wt% | wt% | wt% | wt% | |
| 1028 | 82 | 10 | 0 | 8 | 0 | 0 | Not centrifuged, turbid solution |
| 1029 | 72 | 20 | 0 | 8 | 0 | 0 | Oil separated |
| 1030 | 82 | 0 | 10 | 8 | 0 | 0 | Oil separated |
| 1031 | 72 | 0 | 20 | 8 | 0 | 0 | Oil separated |
| 1032 | 82 | 0 | 0 | 8 | 10 | 0 | Oil separated |
| 1034 | 72 | 0 | 0 | 8 | 0 | 20 | Oil separated |
| 1035 | 52 | 0 | 0 | 8 | 0 | 40 | Oil separated |

**Table 2-2. Comparison of the oil-holding capacities of oleogel compositions with added stabilizers**

| No. | CO | NMP | BUP | MLX | ATO5 | SPC | 9000rpm15min | |
|---|---|---|---|---|---|---|---|---|
| | wt% | wt% | wt% | wt% | wt% | wt% | Standing at 40 °C | Standing at 25 °C |
| 1009 | 68.82 | 10 | 6 | 0.18 | 15 | / | Oil separated | Oil separated |
| 1012 | 58.82 | 10 | 6 | 0.18 | 15 | 10 | No oil separated | No oil separated |

**Table 2-3. Oil-holding capacity study of oleogel compositions with respect to the amount of stabilizers**

| No. | CO | NMP | BUP | MLX | ATO5 | SPC | 9000rpm15min | |
|---|---|---|---|---|---|---|---|---|
| | wt% | wt% | wt% | wt% | wt% | wt% | 40°C | 25°C |
| 1014 | 68.85 | 10 | 5 | 0.15 | 15 | 1 | No oil separated | Oil separated |
| 1015 | 66.85 | 10 | 5 | 0.15 | 15 | 3 | No oil separated | Oil separated |
| 1016 | 64.85 | 10 | 5 | 0.15 | 15 | 5 | No oil separated | No oil separated |
| 1017 | 61.85 | 10 | 5 | 0.15 | 15 | 8 | No oil separated | No oil separated |

**Table 2-4. Oil-holding capacity study of oleogel compositions with respect to the amount of stabilizers**

| No. | CO | DMSO | BUP | MLX | ATO5 | SPC | 9000rpm15min | |
|---|---|---|---|---|---|---|---|---|
| | wt% | wt% | wt% | wt% | wt% | wt% | 40°C | 25°C |
| 1044 | 67.82 | 5 | 6 | 0.18 | 20 | 1 | No oil separated | No oil separated |
| 1045 | 66.82 | 5 | 6 | 0.18 | 20 | 2 | No oil separated | No oil separated |
| 1046 | 65.82 | 5 | 6 | 0.18 | 20 | 3 | No oil separated | No oil separated |

**Table 2-5. Oil-holding capacity study of oleogel compositions with respect to the amount of stabilizers**

| No. | CO | DMSO | BUP | MLX | Geleol | DOPC | 9000rpm15min | |
|---|---|---|---|---|---|---|---|---|
| | wt% | wt% | wt% | wt% | wt% | wt% | 40°C | 25°C |
| 1066 | 67.82 | 5 | 6 | 0.18 | 20 | 1 | No oil separated | No oil separated |
| 1067 | 66.82 | 5 | 6 | 0.18 | 20 | 2 | No oil separated | No oil separated |
| 1068 | 65.82 | 5 | 6 | 0.18 | 20 | 3 | No oil separated | No oil separated |

**Table 2-6. Oil-holding capacity study of oleogel compositions comprising stabilizers**

| No. | CO | BA | BUP | MLX | NMP | ATO5 | DPPC | DMPC | DSPC | HSPC | 14000rpm10min |
|---|---|---|---|---|---|---|---|---|---|---|---|
| | wt% | wt% | wt% | wt% | wt% | wt% | wt% | wt% | wt% | wt% | |
| 1024 | 61.76 | 7.84 | 8 | 0.24 | 2.16 | 15 | 5 | 0 | 0 | 0 | No oil separated |
| 1025 | 61.76 | 7.84 | 8 | 0.24 | 2.16 | 15 | 0 | 5 | 0 | 0 | No oil separated |
| 1026 | 61.76 | 7.84 | 8 | 0.24 | 2.16 | 15 | 0 | 0 | 5 | 0 | No oil separated |
| 1027 | 61.76 | 7.84 | 8 | 0.24 | 2.16 | 15 | 0 | 0 | 0 | 5 | No oil separated |

**Table 2-7. Oil-holding capacity study of oleogel compositions comprising stabilizers**

| No. | CO | BA | DMSO | BUP | MLX | ATO5 | POPC | DEPC | EPC | DOPC | CHO | 9000rpm |
|---|---|---|---|---|---|---|---|---|---|---|---|---|
| | wt% | wt% | wt% | wt% | wt% | wt% | wt% | wt% | wt% | wt% | wt% | 15min |
| 1039 | 61.76 | 5.44 | 4.56 | 8 | 0.24 | 15 | 5 | 0 | 0 | 0 | 0 | No oil separated |
| 1040 | 61.76 | 5.44 | 4.56 | 8 | 0.24 | 15 | 0 | 5 | 0 | 0 | 0 | No oil separated |
| 1041 | 61.76 | 5.44 | 4.56 | 8 | 0.24 | 15 | 0 | 0 | 5 | 0 | 0 | No oil separated |
| 1042 | 61.76 | 5.44 | 4.56 | 8 | 0.24 | 15 | 0 | 0 | 0 | 5 | 0 | No oil separated |
| 1043 | 61.76 | 5.44 | 4.56 | 8 | 0.24 | 15 | 0 | 0 | 0 | 0 | 5 | Marginal oil separated at the edge |

**Table 2-8. Oil-holding capacity study of oleogel compositions comprising stabilizers**

| No. | CO | BA | DMSO | BUP | MLX | GELUCIRE 43/01 | POPC | DEPC | EPC | DOPC | 9000rpm15min |
|---|---|---|---|---|---|---|---|---|---|---|---|
| | wt% | wt% | wt% | wt% | wt% | wt% | wt% | wt% | wt% | wt% | |
| 1069 | 59.7 | 4.3 | 5.7 | 10 | 0.3 | 15 | 5 | 0 | 0 | 0 | No oil separated |
| 1070 | 59.7 | 4.3 | 5.7 | 10 | 0.3 | 15 | 0 | 5 | 0 | 0 | No oil separated |
| 1071 | 59.7 | 4.3 | 5.7 | 10 | 0.3 | 15 | 0 | 0 | 5 | 0 | No oil separated |
| 1072 | 59.7 | 4.3 | 5.7 | 10 | 0.3 | 15 | 0 | 0 | 0 | 5 | No oil separated |

**Table 2-9. Oil-holding capacity study of oleogel compositions comprising stabilizers**

| No. | CO | BA | BUP | Cithrol GMS 40 | SPC | 9000rpm15min |
|---|---|---|---|---|---|---|
| | wt% | wt% | wt% | wt% | wt% | |
| 1073 | 27 | 10 | 3 | 30 | 30 | No oil separated |
| 1075 | 72 | 0 | 3 | 20 | 5 | No oil separated |

Oil-holding capacity is one of the indicators for evaluating the structural stability of an oleogel. In order to ensure the ability of a gelator to solidify a liquid oil to prevent the liquid oil from separating during long-term standing, the oil-holding capacity of the formulation needs to be studied to determine the maximum oil-bearing capacity of the oleogel. The present disclosure unexpectedly revealed that the compositions comprising stabilizers had significantly improved oil-holding capacities and improved physical stability, and thus the risk of oil separation during storage was effectively reduced.

### Example 3

### Viscosity Measurements of Oleogel Compositions

Pharmaceutical compositions comprising different amounts of the stabilizer and organic solvent were prepared according to Tables 3-1 to 3-3. The starting auxiliary materials were mixed at 70 °C, heated with stirring until a transparent and uniform solution was formed, and cooled to room temperature to form a solid gel-like substance. Subsequently, the viscosities of the pharmaceutical compositions were measured through the spindle method using a viscometer equipped with a No. 14 spindle at a temperature of 30 °C at a rotational speed of 10 rpm, and the viscosity measurements are shown in Tables 3-1 to 3-3 below.

**Table 3-1. Viscosity measurements of pharmaceutical compositions comprising no stabilizer**

| No. | CO | BA | BUP | ATO5 | Viscosity |
|---|---|---|---|---|---|
| | wt% | wt% | wt% | wt% | cP |
| 1058 | 79 | 0 | 6 | 15 | 98000 |
| 1059 | 69 | 10 | 6 | 15 | 93375 |

**Table 3-2. Viscosity measurements of pharmaceutical compositions comprising different stabilizers**

| No. | CO | BA | DMSO | NMP | BUP | MLX | ATO5 | DEPC | DOPC | CHO | SPC | Viscosity |
|---|---|---|---|---|---|---|---|---|---|---|---|---|
| | wt% | wt% | wt% | wt% | wt% | wt% | wt% | wt% | wt% | wt% | wt% | cP |
| 1040 | 61.76 | 5.44 | 4.56 | 0 | 8 | 0.24 | 15 | 5 | 0 | 0 | 0 | 5337.5 |
| 1042 | 61.76 | 5.44 | 4.56 | 0 | 8 | 0.24 | 15 | 0 | 5 | 0 | 0 | 6262.5 |
| 1043 | 61.76 | 5.44 | 4.56 | 0 | 8 | 0.24 | 15 | 0 | 0 | 5 | 0 | 5650 |
| 1011 | 63.82 | 0 | 0 | 10 | 6 | 0.18 | 10 | 0 | 0 | 0 | 10 | 5087.5 |

**Table 3-3. Viscosity measurements of pharmaceutical compositions comprising different amounts of the organic solvent**

| No. | CO | DMSO | BA | BUP | MLX | ATO5 | SPC | VE | Viscosity |
|---|---|---|---|---|---|---|---|---|---|
| | wt% | wt% | wt% | wt% | wt% | wt% | wt% | wt% | cP |
| 1056 | 56.61 | 5 | 10 | 8 | 0.24 | 15 | 5 | 0.15 | 2737.5 |
| 1057 | 51.61 | 5 | 15 | 8 | 0.24 | 15 | 5 | 0.15 | 3650 |

The present disclosure unexpectedly revealed that the viscosity of the pharmaceutical composition could be significantly reduced by adding a stabilizer (e.g., SPC, DEPC, CHO, DOPC, etc.), and meanwhile, the amount of the organic solvent also affected the viscosity of the entire system. Therefore, the viscosity of the pharmaceutical composition can be optimized by adjusting the proportions of the stabilizer and the organic solvent, so that the pharmaceutical composition is convenient for clinical administration.

### Example 4

### Syringeability Study

Oleogel compositions comprising different organic solvents and different stabilizers were formulated according to Tables 4-1 to 4-4, and the compositions in the tables were subjected to syringeability tests to determine the maximum pushing forces. The test results are shown in Tables 4-1 to 4-4 below.

**Table 4-1. Syringeability study**

| No. | CO | BA | BUP | ATO5 | Maximum pushing force |
|---|---|---|---|---|---|
| | wt% | wt% | wt% | wt% | kg |
| 1058 | 79 | 0 | 6 | 15 | 0.92 |
| 1059 | 69 | 10 | 6 | 15 | 0.57 |

**Table 4-2. Syringeability study with respect to different amounts of the organic solvent**

| No. | CO | DMSO | BA | BUP | MLX | ATO5 | SPC | VE | Maximum pushing force |
|---|---|---|---|---|---|---|---|---|---|
| | wt% | wt% | wt% | wt% | wt% | wt% | wt% | wt% | kg |
| 1056 | 56.61 | 5 | 10 | 8 | 0.24 | 15 | 5 | 0.15 | 0.5 |
| 1057 | 51.61 | 5 | 15 | 8 | 0.24 | 15 | 5 | 0.15 | 0.41 |
| 1055 | 61.61 | 4.56 | 5.44 | 8 | 0.24 | 15 | 5 | 0.15 | 0.78 |
| 1064 | 37.2 | 15 | 15 | 6 | 1.8 | 20 | 5 | 0 | 0.89 |

**Table 4-3. Syringeability study with respect to different stabilizers**

| No. | CO | BA | DMSO | BUP | MLX | ATO5 | DEPC | DOPC | CHO | Maximum pushing force |
|---|---|---|---|---|---|---|---|---|---|---|
| | wt% | wt% | wt% | wt% | wt% | wt% | wt% | wt% | wt% | kg |
| 1040 | 61.76 | 5.44 | 4.56 | 8 | 0.24 | 15 | 5 | 0 | 0 | 0.67 |
| 1042 | 61.76 | 5.44 | 4.56 | 8 | 0.24 | 15 | 0 | 5 | 0 | 0.56 |
| 1043 | 61.76 | 5.44 | 4.56 | 8 | 0.24 | 15 | 0 | 0 | 5 | 0.47 |

**Table 4-4. Syringeability results of pharmaceutical compositions comprising different amounts of the stabilizer**

| No. | CO | BA | BUP | DMSO | MLX | Cithrol GMS 40 | SPC | VE | Maximum pushing force |
|---|---|---|---|---|---|---|---|---|---|
| | wt% | wt% | wt% | wt% | wt% | wt% | wt% | wt% | kg |
| 1050 | 61.61 | 5.44 | 8 | 4.56 | 0.24 | 15 | 5 | 0.15 | 0.55 |
| 1051 | 51.61 | 5.44 | 8 | 4.56 | 0.24 | 20 | 10 | 0.15 | 0.62 |

Generally, for the ease of administration by doctors, the syringeability in clinical administration requires the maximum pushing force not to be greater than 2 kg.

In the present disclosure, the effect of the amount of the organic solvent on syringeability was studied. The results show that the pushing force can be reduced by increasing the amount of the organic solvent, which is beneficial to clinical administration. In addition, the pushing force can also be reduced by adding other stabilizers to the oleogel composition.

### Example 5

### Rheological Study of Compositions

Oleogel compositions were prepared according to Tables 5-1 to 5-4 and subjected to rheological studies. Rheometer model: TA DHR-1 Sample amount: 1 mL. Measurement mode: oscillation mode: time scanning, fixed strain 0.5%, frequency 1 Hz; oscillation mode: frequency scanning, fixed strain 0.5%, frequency scanning range: 0.1-100 rad/s; flow mode: viscosity scanning, shear rate range: 0.01-100 1/s; flow mode: viscosity scanning at varying temperatures, temperature varying range: 20-60 °C, fixed shear rate: 0.1 1/s.

**Table 5-1. Components of compositions**

| No. | CO | NMP | BUP | MLX | ATO5 | SPC |
|---|---|---|---|---|---|---|
| | wt% | wt% | wt% | wt% | wt% | wt% |
| 1010 | 63.82 | 10 | 6 | 0.18 | 20 | / |
| 1013 | 53.82 | 10 | 6 | 0.18 | 20 | 10 |

**Table 5-2. Components of compositions**

| No. | CO | BA | BUP | DMSO | MLX | Cithrol GMS 40 | SPC | VE |
|---|---|---|---|---|---|---|---|---|
| | wt% | wt% | wt% | wt% | wt% | wt% | wt% | wt% |
| 1051 | 51.61 | 5.44 | 8 | 4.56 | 0.24 | 20 | 10 | 0.15 |
| 1052 | 56.61 | 5.44 | 8 | 4.56 | 0.24 | 20 | 5 | 0.15 |

**Table 5-3. Components of compositions**

| No. | CO | SUPPOCIRE CM | BUP |
|---|---|---|---|
| | wt% | wt% | wt% |
| 1030 | 82 | 10 | 8 |
| 1031 | 72 | 20 | 8 |

**Table 5-4. Components of compositions**

| No. | CO | BA | BUP | DMSO | MLX | ATO5 | SPC | VE |
|---|---|---|---|---|---|---|---|---|
| | wt% | wt% | wt% | wt% | wt% | wt% | wt% | wt% |
| 1053 | 51.61 | 5.44 | 8 | 4.56 | 0.24 | 20 | 10 | 0.15 |
| 1054 | 56.61 | 5.44 | 8 | 4.56 | 0.24 | 20 | 5 | 0.15 |

The parameters storage modulus G' and loss modulus G" in rheology can be used to evaluate gel properties. Theoretically, the larger the storage modulus G' of a glyceride, the greater the gel strength and the more stable the gel network structure. When the storage modulus (G') is greater than the loss modulus (G"), the sample exhibits gel properties. FIGs. 1-1 to 1-7 show the rheological properties of the oleogel compositions in Tables 5-1 to 5-3. The present disclosure unexpectedly revealed that the SPC-comprising composition (1013) in which G' was greater than G" exhibited significant gel properties, while compositions comprising no SPC (1010, 1030 and 1031) did not exhibit gel properties. Meanwhile, the gel strength of the formulation could be improved by increasing the amount of the stabilizer (1051 and 1052).

FIGs. 1-8 and 1-9 show the temperature-dependent trends of the viscosities of compositions 1010, 1013, 1051 and 1052, from which the gelation temperatures of the compositions can be inferred, and the results are shown in Table 5-5. It can be seen from Table 5-5 that the gelation temperature of the composition can be lowered by adding SPC, which is more beneficial to scale-up production and filling.

**Table 5-5. Gelation temperatures of compositions**

| No. | Gelation temperature (°C) |
|---|---|
| 1010 | 49.1 |
| 1013 | 45.2 |
| 1051 | 40.2 |
| 1052 | 40.2 |

FIG. 1-10 shows the rotational speed-dependent trends of the viscosities of compositions 1053 and 1054, from which it can be seen that the oleogel compositions of the present disclosure exhibit shear thinning properties, which is beneficial to clinical administration. Meanwhile, the increase in the amount of the stabilizer SPC led to a certain decrease in the viscosity of the composition.

### Example 6

### Solidification Temperature Study

Oleogel compositions were prepared according to Tables 6-1 to 6-3 and observed under a microscope for the solidification temperatures in cooling the dissolved compositions, and the effects of organic solvents and stabilizers on the solidification temperature were studied. The results are shown in Tables 6-1 to 6-3 below.

**Table 6-1. Solidification temperatures of compositions comprising no stabilizer**

| No. | CO | BA | BUP | ATO5 | Solidification temperature |
|---|---|---|---|---|---|
| | wt% | wt% | wt% | wt% | °C |
| 1058 | 79 | 0 | 6 | 15 | 52 |
| 1059 | 69 | 10 | 6 | 15 | 45 |

**Table 6-2. Solidification temperatures of compositions comprising different organic solvents**

| No. | CO | DMSO | BA | BUP | MLX | ATO5 | SPC | VE | Solidification temperature |
|---|---|---|---|---|---|---|---|---|---|
| | wt% | wt% | wt% | wt% | wt% | wt% | wt% | wt% | °C |
| 1056 | 56.61 | 5 | 10 | 8 | 0.24 | 15 | 5 | 0.15 | 41 |
| 1057 | 51.61 | 5 | 15 | 8 | 0.24 | 15 | 5 | 0.15 | 40 |
| 1049 | 61.61 | 10 | 0 | 8 | 0.24 | 15 | 5 | 0.15 | 44 |
| 1055 | 61.61 | 4.56 | 5.44 | 8 | 0.24 | 15 | 5 | 0.15 | 42 |

**Table 6-3. Solidification temperatures of compositions comprising different stabilizers**

| No. | CO | BA | DMSO | BUP | MLX | ATO5 | POPC | DEPC | EPC | DOPC | CHO | Solidification temperature |
|---|---|---|---|---|---|---|---|---|---|---|---|---|
| | wt% | wt% | wt% | wt% | wt% | wt% | wt% | wt% | wt% | wt% | wt% | °C |
| 1039 | 61.76 | 5.44 | 4.56 | 8 | 0.24 | 15 | 5 | 0 | 0 | 0 | 0 | 43 |
| 1040 | 61.76 | 5.44 | 4.56 | 8 | 0.24 | 15 | 0 | 5 | 0 | 0 | 0 | 38 |
| 1041 | 61.76 | 5.44 | 4.56 | 8 | 0.24 | 15 | 0 | 0 | 5 | 0 | 0 | 38 |
| 1042 | 61.76 | 5.44 | 4.56 | 8 | 0.24 | 15 | 0 | 0 | 0 | 5 | 0 | 40 |
| 1043 | 61.76 | 5.44 | 4.56 | 8 | 0.24 | 15 | 0 | 0 | 0 | 0 | 5 | 44 |

The present disclosure unexpectedly revealed that the solidification temperature of the composition could be lowered by adding an organic solvent and a stabilizer, which was beneficial to production and filling.

### Example 7

### Oleogel Compositions Comprising Different Active Ingredients

Oleogel compositions comprising different main active ingredients were prepared according to each of the components shown in Table 7-1 below. The starting auxiliary materials were mixed at 70 °C, heated with stirring until a transparent and uniform solution was formed, and cooled to room temperature to form a solid gel-like substance.

**Table 7-1. Compositions comprising different active ingredients**

| No. | CO | BA | ROP | DMSO | MLX | Geleol | ATO5 | SPC | VE |
|---|---|---|---|---|---|---|---|---|---|
| | wt% | wt% | wt% | wt% | wt% | wt% | wt% | wt% | wt% |
| 1036 | 66.76 | 5 | 3 | 5 | 0.09 | 15 | 0 | 5 | 0.15 |
| 1037 | 61.76 | 5 | 3 | 5 | 0.09 | 20 | 0 | 5 | 0.15 |
| 1038 | 66.76 | 5 | 3 | 5 | 0.09 | 0 | 15 | 5 | 0.15 |

**Table 7-2. Compositions comprising different active ingredients**

| No. | CO | DMSO | BA | LBUP | TAC | KTL | PRX | ATO5 | SPC |
|---|---|---|---|---|---|---|---|---|---|
| | wt% | wt% | wt% | wt% | wt% | wt% | wt% | wt% | wt% |
| 1060 | 66.91 | 5 | 5 | 3 | 0 | 0 | 0.09 | 15 | 5 |
| 1061 | 67 | 5 | 5 | 0 | 3 | 0 | 0 | 15 | 5 |
| 1062 | 65 | 5 | 5 | 0 | 0 | 5 | 0 | 15 | 5 |

### Example 8

### Pharmaceutical Compositions Comprising Different Concentrations of Active Ingredient and Antioxidant

Oleogel compositions comprising different concentrations of the active ingredient and antioxidant were prepared according to each of the components shown in Table 8 below. The starting auxiliary materials were mixed at 70 °C, heated with stirring until a transparent and uniform solution was formed, and cooled to room temperature to form a solid gel-like substance.

**Table 8. Pharmaceutical compositions comprising different concentrations and antioxidants**

| No. | CO | DMSO | BUP | MLX | ATO5 | SPC | VE | Thioglycerol | BHA | BHT |
|---|---|---|---|---|---|---|---|---|---|---|
| | wt% | wt% | wt% | wt% | wt% | wt% | wt% | wt% | wt% | wt% |
| 1018 | 61.71 | 10 | 8 | 0.24 | 15 | 5 | 0 | 0.05 | 0 | 0 |
| 1022 | 61.71 | 10 | 8 | 0.24 | 15 | 5 | 0 | 0 | 0.05 | 0 |
| 1023 | 61.71 | 10 | 8 | 0.24 | 15 | 5 | 0 | 0 | 0 | 0.05 |
| 1082 | 66.61 | 10 | 8 | 0.24 | 10 | 5 | 0 | 0 | 0 | 0.15 |
| 1019 | 59.55 | 10 | 10 | 0.3 | 15 | 5 | 0.15 | 0 | 0 | 0 |
| 1020 | 63.67 | 10 | 6 | 0.18 | 15 | 5 | 0.15 | 0 | 0 | 0 |

### Example 9. Pharmaceutical Compositions Comprising No Organic Solvent or Comprising Different Organic Solvents

Oleogel compositions comprising different organic solvents were prepared according to each of the components shown in Table 9 below. The starting auxiliary materials were mixed at 70 °C, heated with stirring until a transparent and uniform solution was formed, and cooled to room temperature to form a solid gel-like substance.

**Table 9. Pharmaceutical compositions comprising no organic solvent or comprising different organic solvents**

| No. | CO | DMSO | NMP | EtOH | BA | BUP | MLX | ATO5 | SPC | Thioglycerol | BHT |
|---|---|---|---|---|---|---|---|---|---|---|---|
| | wt% | wt% | wt% | wt% | wt% | wt% | wt% | wt% | wt% | wt% | wt% |
| 1083 | 66.46 | 10 | 0 | 0 | 0 | 8 | 0.24 | 10 | 5 | 0.15 | 0.15 |
| 1084 | 66.61 | 0 | 0 | 0 | 10 | 8 | 0.24 | 10 | 5 | 0.15 | 0 |
| 1085 | 61.46 | 0 | 0 | 10 | 0 | 8 | 0.24 | 15 | 5 | 0.15 | 0.15 |
| 1086 | 59.85 | 0 | 10 | 0 | 0 | 5 | 0.15 | 15 | 10 | 0 | 0 |
| 1087 | 71.46 | 0 | 0 | 0 | 0 | 8 | 0.24 | 15 | 5 | 0.15 | 0.15 |

### Example 10

### In Vivo Administration of Oleogel Compositions

*In vivo* pharmacokinetic studies were carried out in dogs as follows. Beagles weighing about 10 kg were fasted for over 12 h (removing the feeding box) before the experiment, given *ad libitum* access to water, and given food 4 h after administration. Administration was performed to each group by subcutaneous injection at 6 mg/kg. The samples are shown in Table 10. Blood samples of about 0.5 mL were collected from the animals in each group into EDTA-2K+ anticoagulated blood collection tubes at 0 h before the administration, and at 0.5 h, 1 h, 2 h, 3 h, 6 h, 8 h, 12 h, 24 h, 36 h, 48 h, 60 h, 72 h, and 96 h after the administration. Plasma was collected after the whole blood was centrifuged at 8000 rpm for 5 min, and then the drug concentrations in plasma samples were determined by LC-MS/MS.

**Table 10. Formulas of compositions**

| No. | CO | SPC | NMP | DMSO | BUP | MLX | ATO5 |
|---|---|---|---|---|---|---|---|
| | wt% | wt% | wt% | wt% | wt% | wt% | wt% |
| 1047 | 64.85 | 5 | 0 | 10 | 5 | 0.15 | 15 |
| 1048 | 64.85 | 5 | 10 | 0 | 5 | 0.15 | 15 |

The BUP and MLX plasma concentration-time curves within 96 h after administration of the composition are shown in FIGs. 2-1 to 2-4.

### Example 11

### In Vivo Administration of Oleogel Compositions

Pharmacodynamic studies were carried out in rats as follows. The PWT value was measured once a day over three days before the experiment, and once before modeling on the fourth day as a premodeling basal value. Subsequently, 100 µL of CFA was administered to each rat in the right hind paws. The PWT value was measured once again 24 h after inflammation modeling as a post-modeling basal value. Compositions were subcutaneously injected into the footpad. The samples are shown in Table 11. Administration was not performed to the model group. The mechanical paw withdrawal thresholds (PWTs) of the rats were determined as the pain thresholds by irritating the middle part of the footpad of a hind limb with Von Frey monofilaments at different time points after the administration. The time points were 30 min, 1 h, 4 h, 8 h, 12 h, 24 h, 48 h, 72 h and 92 h after the administration.

**Table 11. Samples**

| No. | CO | NMP | BUP | MLX | ATO5 | SPC |
|---|---|---|---|---|---|---|
| | wt% | wt% | wt% | wt% | wt% | wt% |
| 1076 | 54.7 | 10 | 10 | 0.3 | 15 | 10 |
| 1077 | 55 | 10 | 10 | 0 | 15 | 10 |
| 1078 | 60.8 | 11.1 | 0 | 0.3 | 16.7 | 11.1 |

The results are shown in FIG. 3-1. Before the modeling, the mechanical paw withdrawal thresholds (PWTs) of the rats in each group were all kept at 21-24 g; 24 h after the modeling, the mechanical paw withdrawal thresholds (PWTs) decreased to 4-6 g, indicating that the mechanical allodynia of the rats was very significant after the CFA modeling. Composition 1076 significantly increased the PWT value (p<0.05) in rats only 30 min after administration and the effect persisted until hour 96. The difference is of statistical significance (p<0.05). Composition 1078 produced an analgesic effect 4 h after administration (p<0.05), and the analgesic effect persisted until hour 12 and then disappeared (p>0.05), indicating that the effect lasted for a period of time between 12 h and 24 h. Composition 1077 produced no analgesic effect until hour 8 (p<0.05), and the analgesic effect persisted until hour 12, then fluctuated, and appeared again at 48 h (p<0.05).

### Example 12

### In Vivo Administration of Oleogel Compositions

*In vivo* pharmacokinetic studies were carried out in dogs as follows. Beagles weighing about 10 kg were fasted for over 12 h (removing the feeding box) before the experiment, given *ad libitum* access to water, and given food 4 h after administration. Administration was performed to each group by subcutaneous injection at 6 mg/kg. The samples are shown in Table 12. Blood samples of about 0.5 mL were collected from the animals in each group into EDTA-2K+ anticoagulated blood collection tubes at 0 h before the administration, and at 0.5 h, 1 h, 2 h, 3 h, 6 h, 8 h, 12 h, 24 h, 36 h, 48 h, 60 h, 72 h, and 96 h after the administration. Plasma was collected after the whole blood was centrifuged at 8000 rpm for 5 min, and then the drug concentrations in plasma samples were determined by LC-MS/MS.

**Table 12. Formulas of compositions**

| No. | CO | VES | SPC | ATO 5 | BA | ROP | MLX | NMP |
|---|---|---|---|---|---|---|---|---|
| | wt% | wt% | wt% | wt% | wt% | wt% | wt% | wt% |
| 1079 | 30.46 | 18.2 | 27.3 | 9.1 | 9.1 | 4.5 | 0.14 | 1.2 |

The ROP and MLX plasma concentration-time curves within 96 h after administration of the composition are shown in FIGs. 4-1 to 4-2.

### Example 13

### In Vivo Administration of Oleogel Compositions

*In vivo* pharmacokinetic studies were carried out in dogs as follows. Beagles weighing about 10 kg were fasted for over 12 h (removing the feeding box) before the experiment, given *ad libitum* access to water, and given food 4 h after administration. Administration was performed to each group by subcutaneous injection at 6 mg/kg. The samples are shown in Table 13. Blood samples of about 0.5 mL were collected from the animals in each group into EDTA-2K+ anticoagulated blood collection tubes at 0 h before the administration, and at 0.5 h, 1 h, 2 h, 3 h, 6 h, 8 h, 12 h, 24 h, 36 h, 48 h, 60 h, 72 h, and 96 h after the administration. Plasma was collected after the whole blood was centrifuged at 8000 rpm for 5 min, and then the drug concentrations in plasma samples were determined by LC-MS/MS.

**Table 13. Formulas of compositions**

| No. | CO | SPC | BA | DMSO | MLX | ATO5 | Benzoic acid | Maleic acid | ROP |
|---|---|---|---|---|---|---|---|---|---|
| | wt% | wt% | wt% | wt% | wt% | wt% | wt% | wt% | wt% |
| 1080 | 59.82 | 5 | 4 | 6 | 0.18 | 15 | 4 | 0 | 6 |
| 1081 | 59.82 | 5 | 4 | 6 | 0.18 | 15 | 0 | 4 | 6 |

The ROP and MLX plasma concentration-time curves within 96 h after administration of the composition are shown in FIGs. 5-1 to 5-4.

The exemplary embodiments of the present disclosure have been described above. However, the scope of the present disclosure is not limited to the above embodiments. Any modification, equivalent, improvement and the like made without departing from the spirit and principle of the present disclosure shall fall within the protection scope of the present disclosure.

## Claims

1. A pharmaceutical composition, comprising the following components:
a. a liquid oil
b. a pharmaceutically acceptable gelator, selected from one or more of fatty acid glycerides of formula I: 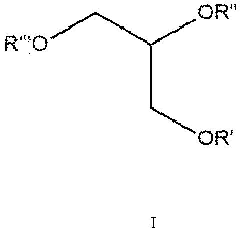 wherein R' and R" may be identical or different and are each independently selected from H or RaCO, and R‴ is selected from RaCO; each Ra is independently selected from saturated or unsaturated aliphatic hydrocarbyl;
c. a pharmaceutically acceptable stabilizer, selected from one or more of compounds of formula II or formula III: the compound of formula II being 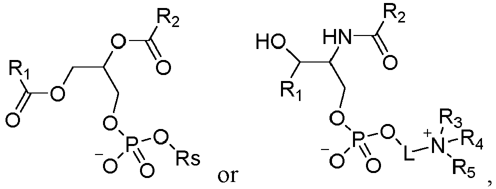 wherein Rs is selected from H, 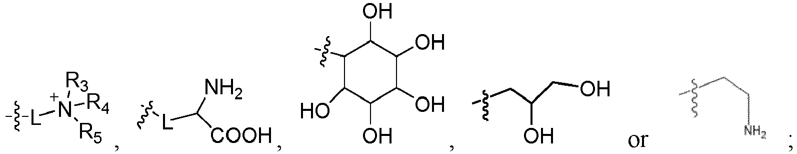 R₁ and R₂ are identical or different and are each independently selected from saturated or unsaturated aliphatic hydrocarbyl; R₃, R₄ and R₅ are identical or different and are each independently selected from H or alkyl; L is selected from alkylene; the compound of formula III being 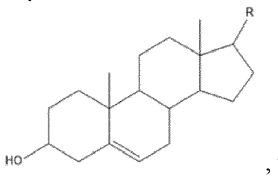 wherein R is selected from alkyl; and
d. at least one pharmaceutically active ingredient.

2. The pharmaceutical composition according to claim 1, wherein in the formula I, each Ra is independently selected from alkyl; preferably, each Ra is independently selected from C₁₋₄₀ alkyl; more preferably, each Ra is independently selected from C₇₋₄₀ alkyl;
preferably, R' and R" are selected from H, and R‴ is selected from (C₁₁₋₄₀ alkyl)C(=O); or, R' is selected from H, R" is selected from RaCO, and a total number of carbon atoms in the Ra alkyl selected for each of R" and R‴ independently is greater than 18; or, R', R" and R‴ are all selected from RaCO, and a total number of carbon atoms in the Ra alkyl selected for each of R', R" and R‴ independently is greater than 17;
preferably, in the formula II, R₁ and R₂ are identical or different and are each independently selected from C₁₀₋₃₀ saturated or unsaturated aliphatic hydrocarbyl, e.g., C₁₃₋₂₁ alkyl; R₃, R₄ and R₅ are identical or different and are each independently selected from H or C₁₋₁₀ alkyl, for example, from H, methyl or ethyl; L is selected from C₁₋₁₀ alkylene; preferably, L is selected from C₁₋₆ aklylene, and for example, is methylene or ethylidene; in the compound of formula III, R is selected from C₁₋₁₀ alkyl, e.g., C₈₋₁₀ alkyl.

3. The pharmaceutical composition according to claim 1 or 2, wherein
the component b pharmaceutically acceptable gelator includes, for example, one or more of glyceryl monostearate, glyceryl distearate, glyceryl tristearate, glyceryl monobehenate, glyceryl dibehenate, glyceryl monopalmitate stearate, glyceryl dipalmitate stearate, glyceryl monopalmitate, glyceryl dipalmitate and glyceryl palmitate stearate;
the component c pharmaceutically acceptable stabilizer is selected from one or more of HSPC (hydrogenated soybean phosphatidylcholine), DMPC (dimyristoylphosphatidylcholine), DPPC (dipalmitoylphosphatidylcholine), DSPC (distearoylphosphatidylcholine), DLPC (dilauroylphosphatidylcholine), SPC soybean phosphatidylcholine (soybean phospholipid), EPC (egg yolk phospholipid), rapeseed phospholipid, sunflower phospholipid, DEPC (dierucoylphosphatidylcholine), DOPC (dioleoylphosphatidylcholine), POPC (palmitoyloleoylphosphatidylcholine), sphingomyelin, distearoyl phosphatidic acid (DSPA), dioleoylphosphatidylethanolamine (DOPE), dipalmitoyl phosphatidic acid (DPPA), myristoyl lysophosphatidylcholine (M-lysoPC), palmitoyl lysophosphatidylcholine (P-lysoPC), 1-stearoyl lysophosphatidylcholine (S-lysoPC), dipalmitoylphosphatidylethanolamine (DPPE), distearoylphosphatidylethanolamine (DSPE), dioleoylphosphatidylglycerol (DOPG), dimyristoylphosphatidylethanolamine (DMPE), dimyristoylphosphatidylglycerol (DMPG), dipalmitoylphosphatidylglycerol (DPPG), 1-palmitoyl-2-oleoylphosphatidylglycerol (POPG), distearoylphosphatidylglycerol (DSPG), dipalmitoylphosphatidylserine (DPPS), phosphatidylinositol (PI) and cholesterol (CHO).

4. The pharmaceutical composition according to any one of claims 1 to 3, wherein the pharmaceutical composition further comprises e. at least one pharmaceutically acceptable solvent;
and/or the pharmaceutical composition may further comprise g. a pharmaceutically acceptable release modifier;
and/or the pharmaceutical composition may further comprise f. a pharmaceutically acceptable acid.

5. The pharmaceutical composition according to any one of claims 1 to 3, wherein the liquid oil is selected from one or a combination of more of castor oil, sesame oil, corn oil, soybean oil, olive oil, safflower oil, cottonseed oil, peanut oil, fish oil, tea oil, almond oil, babassu oil, blackcurrant seed oil, borage oil, canola oil, palm oil, palm kernel oil, sunflower oil, medium-chain triglyceride, glyceryl dioleate and glyceryl monooleate; the at least one pharmaceutically active ingredient is not limited to a therapeutic type, and may be an anti-inflammatory drug, a local anesthetic, an analgesic, an anti-psychiatric drug, an anxiolytic, a sedative-hypnotic drug, an antidepressant, an antihypertensive drug, a steroid hormone, an antiepileptic drug, an antiseptic, an anticonvulsant, an anti-parkinson drug, a central nervous stimulant, an antipsychotic, an antiarrhythmic, an antianginal, an antithyroid drug, an antidote, an antiemetic, a hypoglycemic drug, an anti-tubercular drug, an anti-HIV drug, an anti-HBV drug, an antineoplastic, an anti-rejection drug or a mixture thereof.

6. The pharmaceutical composition according to claim 4, wherein the pharmaceutically acceptable release modifier is selected from small-molecule esters and surfactants; the small-molecule esters are glyceryl triacetate, isopropyl stearate, isopropyl laurate, isopropyl palmitate, isopropyl myristate and benzyl benzoate; the pharmaceutically acceptable acid is selected from acetic acid, lactic acid, succinic acid, fumaric acid, maleic acid, methanesulfonic acid, linoleic acid, sorbic acid, caprylic acid, pelargonic acid, lauric acid, palmitic acid, oleic acid, hydrochloric acid, phthalic acid, capric acid, myristic acid, propionic acid, butyric acid, heptanoic acid, valeric acid, malic acid, tartaric acid, oxalic acid, citric acid, ascorbic acid, salicylic acid, caffeic acid and vitamin E succinic acid.

7. The pharmaceutical composition according to any one of claims 1 to 6, wherein the liquid oil accounts for about 20% to about 90% (w/w) of a total amount of the composition; the gelator accounts for 2% to 50% (w/w) of the total amount of the composition; the stabilizer accounts for 1% to 40% (w/w) of the total amount of the composition; the pharmaceutically active ingredient accounts for 0.01% to 50.0% (w/w) of the total amount of the composition; the total amount of the solvent accounts for 0% to 50% (w/w) of the total amount of the composition.

8. A preparation method for the pharmaceutical composition according to any one of claims 1 to 7, comprising the following steps:
(a1) mixing a liquid oil, a pharmaceutically acceptable gelator, a pharmaceutical stabilizer and a solvent, and stirring under a heating condition to obtain a clear and uniform mixed solution;
(a2) adding at least one pharmaceutically active ingredient to the mixed solution, and stirring to form a uniform mixture; and
(a3) cooling the uniform mixture formed in (a2) to room temperature.
According to an embodiment of the present disclosure, the mixing in step (a1) further comprises adding at least one release modifier; for example, step (a1) may be mixing a liquid oil, a gelator, a pharmaceutical stabilizer and a pharmaceutically acceptable solvent with a release modifier.

9. A sustained-release formulation comprising the pharmaceutical composition according to any one of claims 1 to 7, wherein the formulation is administered as a depot formulation; preferably, the formulation is injectable or the formulation can be administered topically.

10. The formulation according to claim 9, further comprising packaging filled with the formulation, the packaging being selected from one or more of a vial, a pre-filled syringe and a cartridge.
